# EUROPÄISCHE PATENTANMELDUNG

(11) **EP 2 377 556 A1**
(43) Veröffentlichungstag der Anmeldung: **19.10.2011**
(21) Anmeldenummer: 11152416.1
(22) Anmeldetag: 27.01.2011
(51) Int. Cl.: A61K 47/10, A61K 31/00

(54) **Eine Zusammensetzung**

(30) Priorität: 14.04.2010 EP 10003932
(71) Anmelder: Cognis IP Management GmbH, 40589 Düsseldorf (DE)
(72) Erfinder: Petersen, Kirsten, 24118 Kiel (DE); Müller, Bernd, 24220 Flintbek (DE); Rosen, Thomas, 41468 Neuss (DE)
(74) Vertreter: Fabry, Bernd

(57) **Zusammenfassung**

Die vorliegende Erfindung betrifft die Verwendung eines Solubilisators zur Erhöhung der Löslichkeit eines pharmazeutischen Wirkstoffes in Wasser. Weiterhin betrifft die vorliegende Erfindung eine Zusammensetzung enthaltend einen Solubilisator und mindestens einen pharmazeutischen Wirkstoff. In beiden Fällen ist der Solubilisator ein alkoxylierter Monoalkohol oder ein Gemisch alkoxylierter Monoalkohole, wobei der alkoxylierte Monoalkohol oder, wenn ein Gemisch alkoxylierter Monoalkohole vorliegt, mindestens ein alkoxylierter Monoalkohol in dem Gemisch, mindestens eine Oxypropyleneinheit enthält.

## Beschreibung

Die vorliegende Erfindung betrifft die Verwendung eines Solubilisators zur Erhöhung der Löslichkeit eines pharmazeutischen Wirkstoffes in Wasser. Weiterhin betrifft die vorliegende Erfindung eine Zusammensetzung enthaltend einen Solubilisator und mindestens einen pharmazeutischen Wirkstoff. In beiden Fällen ist der Solubilisator ein alkoxylierter Monoalkohol oder ein Gemisch alkoxylierter Monoalkohole, wobei der alkoxylierte Monoalkohol oder, wenn ein Gemisch alkoxylierter Monoalkohole vorliegt, mindestens ein alkoxylierter Monoalkohol in dem Gemisch, mindestens eine Oxypropyleneinheit enthält.

Ein Solubilisator ist ein Stoff oder ein Stoffgemisch, das dazu dient, die Löslichkeit einer in Wasser nur begrenzt oder gar schwer löslichen Verbindung zu erhöhen.

Alkoxylierte Monoalkohole, insbesondere Fettalkoholpolyglycolether sind bekannte Tenside. Sie werden unter anderem im kosmetischen Bereich, unter anderem als Solubilisatoren, eingesetzt. Fettalkoholpolyglycolether werden beschrieben in dem Chemielexikon Römpp Online, Version 3.6 (Georg Thieme Verlag; http://www.roempp.com**)**. Fettalkohole sind lineare, gesättigte oder ungesättigte primäre Alkohole mit 6 bis 22 C-Atomen.

Die Verwendung von ethoxylierten Monoalkoholen, die keine Oxypropyleneinheit enthalten, als Solubilisatoren für pharmazeutische Wirkstoffe ist bekannt, zum Beispiel aus European Journal of Pharmaceutics and Biopharmaceutics, Band 51, Seiten 221 - 226 (2001) und aus International Journal of Pharmaceutics, Band 92, Seiten 191 - 196 (1993) und aus J. Chem. Eng. Data, Band 53, Seiten 1271 - 1277 (2008) und aus Journal of Pharmaceutical Science and Technology, Band 52, Seiten 33 - 36 (1998).

US 2009/311195 offenbart, insbesondere in Anspruch 1, verschiedene Zusammensetzungen enthaltend ein Tensid und eine Aktivkomponente. Eine spezifische Zusammensetzung enthaltend einen alkoxylierten Monoalkohol, der mindestens eine Oxypropyleneinheit enthält, und einen pharmazeutischen Wirkstoff wird nicht offenbart.

Eine Möglichkeit der Benennung von Fettalkoholpolyglycolethern soll an dem folgenden Beispiel erläutert werden. Polyoxypropylen-1-polyoxyethylen-9-laurylether ist erhältlich durch Ethoxylierung und Propoxylierung von Laurylalkohl (1-Dodecanol) und hat im Mittel (Zahlenmittel) eine Oxypropyleneinheit und neun Oxyethyleneinheiten. Dabei kann die Alkoxylierung durch ein Gemisch aus Ethylenoxid und Propylenoxid erfolgen oder sie kann zweistufig durch Alkoxylierung erst mit Ethylenoxid, dann mit Propylenoxid oder umgekehrt erfolgen oder sie kann mehrstufig erfolgen. D. h., dass der Name Polyoxypropylen-1-polyoxyethylen-9-laurylether lediglich besagt, dass pro Molekül im Mittel eine Oxypropyleneinheit und neun Oxyethyleneinheiten vorliegen ohne das gesagt wird in welcher Reihenfolge diese Einheiten im Molekül vorliegen.

Immer mehr Wirkstoffe auf dem pharmazeutischen Markt weisen eine schlechte Wasserlöslichkeit und damit einhergehend häufig auch eine schlechte Bioverfügbarkeit auf. Um diese Wirkstoffe in angemessener Form applizieren zu können, ist es nötig, sie durch unterschiedliche Methoden besser in Lösung zu bringen. Gerade im Bereich der flüssigen Darreichungsformen sind aber zusätzliche Anforderungen, wie möglichst geringe Applikationsvolumina für ausreichende Mengen an Arzneistoff und geringe Toxizität, häufig schwer zu erfüllen. Aus diesem Grund stellt die Lösungsvermittlung/Solubilisierung einen wichtigen Bereich in der Entwicklung neuer Arzneiformen dar.

Es existiert eine Vielzahl chemischer und technologischer Maßnahmen, um die Löslichkeit von Wirkstoffen zu verändern. Eine dieser Methoden ist die Solubilisation von schwerlöslichen Wirkstoffen, bei der oberflächenaktive Substanzen zur Löslichkeitsverbesserung eingesetzt werden. Diese oberflächenaktive Substanzen bestehen aus hydrophilen und lipophilen Molekülteilen, die meistens in getrennten Molekülbereichen vorliegen. Sie werden daher auch als amphiphile Substanzen bezeichnet. Die Tensidmoleküle lagern sich zu so genannten Mizellen zusammen, die im Inneren einen lipophilen Bereich haben, nach außen allerdings hydrophil sind. In diesem lipophilen Innenraum können lipophile Stoffe gehalten werden, sodass sich klare Lösungen bilden.

Auf dem Gebiet der Solubilisation von Wirkstoffen ist eine Reihe von Unternehmen tätig. Hierzu gehören Hersteller von pharmazeutischen Hilfsstoffen wie BASF, Gattefossé, Croda oder Evonik. Dabei werden in der pharmazeutischen Industrie in der Regel nur eine begrenzte Auswahl von bereits in den verschiedenen Arzneimittelbüchern monographierten Solubilisatoren eingesetzt. Demgegenüber gibt es eine Reihe von Solubilsatoren und synergistischer Mischungen von Solubilisatoren für kosmetische Anwendung.

Wie beschrieben stellen schwerlösliche Wirkstoffe ein großes Problem in der pharmazeutischen Entwicklung dar, da diese Stoffe in der Regel schlecht bioverfügbar sind und somit ihre Wirksamkeit z. T. extrem erniedrigt ist. Es gibt eine begrenzte Auswahl von löslichkeitserhöhenden Stoffen, die die Bioverfügbarkeit von solchen schwerlöslichen Stoffen erhöhen können. In vielen Fällen ist diese Auswahl aber nicht ausreichend, um die Löslichkeit von neuen Wirkstoffen so zu erhöhen, dass sie therapeutisch eingesetzt werden können. Ein weiteres Problem sind zudem teilweise toxische Eigenschaften von Solubilisatoren.

Der vorliegenden Erfindung lag die Aufgabe zu Grunde, weitere Solubilisatoren für pharmazeutische Wirkstoffe bereit zu stellen.

Diese Aufgabe wurde gelöst durch die Bereitstellung der in den Patentansprüchen definierten Solubilisatoren zur Solubilisierung pharmazeutischer Wirkstoffe.

Ein Gegenstand der vorliegenden Erfindung ist also die Verwendung eines Solubilisators zur Erhöhung der Löslichkeit eines pharmazeutischen Wirkstoffes in Wasser wie dies in den Patentansprüchen definiert ist.

Ein weiterer Gegenstand der vorliegenden Erfindung ist eine Zusammensetzung wie sie in den Patentansprüchen definiert ist.

Die abhängigen Patentansprüche geben besondere Ausführungsformen der vorliegenden Erfindung wider.

Die erfindungsgemäß zu verwendenden alkoxylierten Monoalkohole können nur einen Fettalkoholrest, z. B. den Laurylrest, oder ein Gemisch verschiedener Fettalkoholreste haben. Insbesondere können zur Alkoxylierung Fettalkohole auf Basis natürlicher Rohstoffe verwendet werden. Zum Beispiel kann so genannter Cocoylalkohol verwendet werden. Cocoylalkohol ist ein Fettalkoholgemisch, das aus Kokosfett erhältlich ist, und das zum größten Teil aus Laurylalkohol besteht.

Erfindungsgemäß kann die Alkoxylierung mit einem Gemisch mehrerer Alkoxide entweder bedeuten, dass ein Alkohol oder ein Alkoholgemisch mit einem Gemisch mehrerer Alkoxide umgesetzt wird oder dass ein Alkohol oder ein Alkoholgemisch erst mit einem Alkoxid umgesetzt wird und das dabei erhältliche Reaktionsprodukt dann mit einem zweiten und danach ggf. mit einem dritten Alkoxid umgesetzt wird.

Es wurde gefunden, dass die erfindungsgemäßen alkoxylierten Monoalkohole, die mindestens eine Oxypropyleneinheit enthalten, bessere Solubilisatoren sind als die aus dem Stand der Technik bekannten alkoxylierten Monoalkohole, bei denen es sich zum Beispiel um ethoxylierte Laurylalkohle handelt, die keine Oxypropyleneinheit enthalten.

### Beispiele

%-Angaben bedeuten jeweils Gew.-%, wenn nichts anderes angegeben ist.

Es wurde eine Reihe amphiphiler Substanzen, die bislang nur in kosmetischen Formulierungen zur Anwendung kommen, hinsichtlich der Anwendung als Solubilisatoren für pharmazeutische Wirkstoffe untersucht. Diese Solubilisatoren wurden bislang nicht für die Erhöhung der Löslichkeit von pharmazeutischen Wirkstoffen verwendet.

Es handelt sich um folgende Produkte:

| | |
|---|---|
| Eumulgin^{®} ES: | PPG-5-Laureth-5 (INCI-Name) |
| Eumulgin^{®} L: | PPG-1-PEG-9 Lauryl Glycol Ether (INCI-Name) |
| Eumulgin^{®} HPS: | Gemisch aus 25-50 % Coceth-7,25-50 % Eumulgin^{®} L, 10-20 % Eumulgin^{®} HRE PH, und 5-10 % Wasser |

Die Struktur der genannten Produkte wird durch die folgende Benennung deutlich:

| | |
|---|---|
| Eumulgin^{®} ES: | Polyoxypropylen-5 Polyoxyethylen-5 Laurylether |
| Eumulgin^{®} L: | Polyoxypropylen-1 Polyoxyethylen-9 Laurylether |
| Eumulgin^{®} HPS: | 25-50 % Polyoxyethylen-7 Cocoylether, 25-50 % Polyoxypropylen-1 Polyoxyethylen-9 Laurylether, 10-20% Polyoxyethyleneglycerol-tri-hydroxystearat 40 und 10-20 % Wasser |

Diese Produkte sind alle erhältlich von der Cognis GmbH, Monheim, Deutschland.

Dabei wurden folgende Ergebnisse gefunden:
- Alle genannten Solubilisatoren lassen sich technisch in pharmazeutischen Formulierungen verwenden. Sie weisen physikalische Eigenschaften (Viskosität, CMC und Mizellgröße) auf, die einen unproblematischen Einsatz vermuten lassen.
- Eumulgin^{®} HPS: Diese synergistische Mischung verschiedener Produkte führte zu einer deutlichen Lösungsverbesserung von verschiedenen Wirkstoffen. Die erhöhten Löslichkeiten liegen in vielen Fällen über der von anderen am Markt etablierten Solubilisatoren.
- Eumulgin^{®} HPS führt zu deutlich höheren Löslichkeitsverbesserungen von Diazepam, die über der von anderen marktüblichen Produkten wie Solutol^{®} HS15 (BASF SE, Ludwigshafen, Deutschland), Eumulgin^{®} RO 35 PH oder Polysorbat^{®} 80 liegt.
- Eumulgin^{®} L ist ein deutlich effektiverer Solubilisator für Estradiol als andere marktübliche Produkte wie Solutol^{®} HS15 (BASF), Eumulgin^{®} RO 35 PH oder Polysorbat^{®} 80.
- Eumulgin^{®} ES ist ein deutlich effektiverer Solubilisator für verschiedene Zimtsäurederivate wie Butylcinnamat und Phenethylcinnamat als andere marktübliche Produkte wie Solutol^{®} HS15 (BASF), Eumulgin^{®} RO 35 PH oder Polysorbat^{®} 80.
- Für pharmazeutische Anwendungen ist zudem relevant, dass alle Solubilisatoren die Löslichkeit der Modellverbindungen in Wasser erhöhten. Die erreichten Konzentrationen gehen über die in Marktprodukten enthaltene Konzentration hinaus. Dies ist relevant, da so Cosolventien wie Ethanol vermieden werden können, die irritierend wirken können und andere toxische Nebenwirkungen insbesondere bei der Anwendung bei Kindern haben können.
- Alle Solubilisatoren sind bereits in kosmetischen Anwendungen etabliert und durch eine hohe Verträglichkeit und geringe Toxizität gekennzeichnet.

Die genannten Produkte und synergistischen Mischungen wurden auf ihre Einsetzbarkeit in pharmazeutischen Formulierungen und insbesondere zur Löslichkeitsverbesserung von schwerlöslichen Modellsubstanzen untersucht. Als Wirkstoffe wurden dabei folgende Stoffe verwendet: Diazepam, Erythromycin, Estradiol, Itraconazol. Die ausgewählten Modellarzneistoffe weisen eine schlechte Wasserlöslichkeit auf. Um ein möglichst breites Spektrum an Arzneistoffen abzudecken, stammen sie aus chemisch unterschiedlichen Gruppen und sind häufig verwendete Wirkstoffe.

Der Einfluss der genannten neuen Solubilisatoren auf die Verstärkung der Löslichkeit dieser Wirkstoffe wurde untersucht und mit bereits für pharmazeutische Anwendungen etablierten Solubilisatoren verglichen. Dazu wurden Lösungsisothermen aufgenommen, mit deren Hilfe man feststellen kann, wie stark die Lösungsvermittlung durch die verschiedenen Solubilisatoren ausgeprägt ist. Optimal ist eine starke Erhöhung der Arzneistoffkonzentration durch den Einsatz geringer Mengen Solubilisator, da mit steigender Konzentration auch die unerwünschten Effekte steigen können.

Die folgende Tabelle gibt die Erhöhung der Löslichkeit verschiedener schwerlöslicher Wirkstoffe durch eine Reihe von Solubilisatoren wieder. Dabei wurden als Modellsubstanzen Diazepam, Erythromycin, Itraconazol und Estradiol ausgewählt. Die untersuchten Solubilisatoren wurden mit bereits für pharmazeutische Formulierungen etablierten Solubilisatoren wie TEGO^{®} SMO 80 V (Polysorbat 80) von der Firma Evonik, Solutol^{®} HS15 von der Firma BASF, Ludwigshafen sowie Eumulgin^{®} RO 35 PH und Speziol^{®} TPGS Pharma (Tocophersolan) von der Firma Cognis, Monheim verglichen.

| | **Ohne Solubilisator** | **TEGO^{®} SMO 80 V** | **Solutol® HS 15** | **Eumulgin** ® **L** | **Eumulgin ® HPS** | **Eumulgin ® RO 35** | **Speziol^{®} TPGS** | **Eumulgin ® ES** |
|---|---|---|---|---|---|---|---|---|
| | mg/ml | mg/ml | mg/ml | mg/ml | mg/ml | mg/ml | mg/ml | mg/ml |
| Diazepam | 0,012 | 1,817 | 1,088 | 1,866 | 2,036 | 1,765 | 1,854 | |
| Erythromycin | 0,459 | 1,695 | | 1,066 | 1,226 | 1,662 | 1,715 | |
| Itraconazol | 0,001 | 0,015 | 0,010 | 0,007 | 0,008 | 0,011 | 0,008 | 0,009 |
| Estradiol | 0,021 | 0,523 | 0,581 | 0,939 | 0,664 | 0,465 | 0,466 | 0,693 |

Untersucht wurde zudem die Solubilisierung einer homologen Reihe von Zimtsäurederivaten. Zimtsäurederivate sind Modellsubstanzen für Wirkstoffe, wie sie z.B. in Sonnenschutzcremes verwendet werden. Um eine breite Auswahl von Molekülen mit unterschiedlicher Molekülgröße zu untersuchen, wurden die folgenden sechs Zimtsäurederivate ausgewählt: Methylcinnamat, Ethylcinnamat, Isopropylcinnamat, Isobutylcinnamat, Benzylcinnamat, und Phenethylcinnamat. Alle verwendeten Zimtsäurederivate sind in Wasser praktisch unlöslich. Durch den Einsatz von Solubilisatoren konnte die Löslichkeit in Wasser deutlich erhöht werden. Als besonders effektive erwiesen sich bei großen und lipophilen Wirkstoffmolekülen Eumulgin^{®} HPS und Eumulgin^{®} ES.

Die folgende Tabelle gibt die löslichkeitverstärkende Wirkung von verschiedenen Solubilisatoren für eine Reihe von Zimtsäurederivaten wieder. Zimtsäurederivate finden vor allem als Lichtschutzfaktoren in Sonnenschutzcremes Verwendung. Die Schwerlöslichkeit dieser Substanzen ist dabei häufig ein Problem, das mithilfe von Solubilisatoren gelöst werden kann.

| **MW [mg/ml]** | **Methyl-cinnamat** | **Ethyl-cinnamat** | **Isopropyl-cinnamat** | **Isobutyl-cinnamat** | **Butyl-cinnamat** | **Phenethyl-cinnamat** |
|---|---|---|---|---|---|---|
| Eumulgin^{®} HPS | 11,4 | 23,6 | 25,7 | 29,0 | 6,4 | 2,7 |
| Eumulgin^{®} ES | 3,0 | | 3,4 | 1,2 | 2,9 | 3,2 |
| Eumulgin^{®} L | 9,8 | 14,7 | 15,2 | 41,0 | 5,8 | 2,5 |
| Eumulgin^{®} O10 | 9,1 | 47,0 | 32,6 | 65,9 | 8,1 | 3,7 |
| Eumulgin^{®} RO35 | 21,5 | 68,0 | 50,9 | 54,2 | 15,3 | 4,9 |
| Speziol^{®} TPGS | 7,9 | 42,3 | 21,0 | 23,1 | 6,1 | 2,6 |
| Tego^{®} SMO 80V | 9,6 | 96,4 | 52,5 | 110,0 | 6,8 | 2,5 |

Der folgende Absatz fasst die Versuchsergebnisse zusammen.

Eumulgin^{®} HPS ist der beste Solubilisator für Diazepam. Diazepam ist ein Wirkstoff aus der Gruppe der Benzodiazepine. Benzodiazepine sind Schlaf- und Beruhigungsmittel. Diazepam hat (aufgrund des aktiven Metaboliten Desmethyldiazepam) eine lange Halbwertszeit und wird vor allem zur Anxiolyse, zur Sedierung und bei Krampfanfällen eingesetzt. In der Roten Liste befinden sich derzeit 13 Präparate, die Diazepam als Wirkstoff enthalten. Sie beinhalten sowohl Lösungen (zur Injektion, zur rektalen Anwendung, zur oralen Anwendung) als auch feste Arzneiformen (Tabletten, Suppositorien). Die Konzentrationen flüssiger Lösungen liegen zwischen 2 mg/ml und 10 mg/ml (RoteListe^{®}-Präparate). Dabei werden in der Regel Cosolventien wie Ethanol und Ethanol/Propylenglycolmischungen verwendet. Diazepam ist in Wasser sehr schwer löslich und hat eine theoretische Löslichkeit von 12,2 µg/ml. Durch die Verwendung von Eumulgin^{®} HPS konnte die Löslichkeit deutlich verbessert werden. So wurde eine maximale Sättigungslöslichkeit in einer 15 %-igen Eumulgin^{®} HPS-Lösung in Wasser von 2,68 mg/ml ermittelt. Dieser Wert ist deutlich höher als für am Markt etablierte Solubilisatoren wie TEGO^{®} SMO 80 V (1,89 mg/mL), Solutol^{®} HS 15 (1,64 mg/mL), Eumulgin^{®} RO35 PH (2,14 mg/ml) und Speziol^{®} TPGS Pharma (2,23 mg/mL) bei gleichen Solubilisatorkonzentrationen.

## Patentansprüche

1. Die Verwendung eines Solubilisators zur Erhöhung der Löslichkeit eines pharmazeutischen Wirkstoffes in Wasser,
wobei der Solubilisator ein alkoxylierter Monoalkohol oder ein Gemisch alkoxylierter Monoalkohole ist, und wobei der Monoalkohol bzw. die Monoalkohole 6 bis 30 C-Atome haben und linear oder verzweigt sind und gesättigt oder ungesättigt sind und primäre, sekundäre oder tertiäre Alkohole sind, und wobei der bzw. die alkoxylierten Monoalkohole durch Alkoxylierung mit Ethylenoxid, Propylenoxid, Butylenoxid oder einem Gemisch aus zwei oder drei der genannten Alkoxide erhältlich sind, und wobei der alkoxylierte Monoalkohol oder, wenn ein Gemisch alkoxylierter Monoalkohole vorliegt, mindestens ein alkoxylierter Monoalkohol in dem Gemisch, mindestens eine Oxypropyleneinheit enthält.

2. Die Verwendung nach Anspruch 1,
wobei der Monoalkohol bzw. die Monoalkohole 6 bis 22, bevorzugt 8 bis 18 und insbesondere 12, C-Atome haben.

3. Die Verwendung nach einem der Ansprüche 1 bis 2,
wobei der Monoalkohol bzw. die Monoalkohole gesättigt oder ungesättigt sind und linear sind und primäre Alkohole sind.

4. Die Verwendung nach einem der Ansprüche 1 bis 3,
wobei der bzw. die alkoxylierten Monoalkohole durch Alkoxylierung mit Ethylenoxid, Propylenoxid oder einem Gemisch aus beiden Alkoxiden erhältlich sind.

5. Die Verwendung nach einem der Ansprüche 1 bis 4,
wobei der bzw. die alkoxylierten Monoalkohole einen mittleren Alkoxylierungsgrad (Zahlenmittel) von 5 bis 20, bevorzugt von 7 bis 10 und insbesondere von 10, haben.

6. Die Verwendung nach einem der Ansprüche 1 bis 4,
wobei der bzw. die alkoxylierten Monoalkohole ausgewählt sind aus der Gruppe bestehend aus Polyoxypropylen-n-laurylether, Polyoxypropylen-m-polyoxyethylen-u-laurylether, und Mischungen aus zwei oder mehr dieser Laurylether,
wobei n = 5 bis 20, bevorzugt 7bis 10, insbesondere 10 ist,
und wobei die Summe aus m und u gleich 5 bis 20, bevorzugt 7bis 10, insbesondere 10 ist,
und wobei u gleich 1 oder größer als 1 ist.

7. Die Verwendung nach einem der Ansprüche 1 bis 4,
wobei der bzw. die alkoxylierten Monoalkohole ausgewählt sind aus der Gruppe bestehend aus Polyoxypropylen-1-polyoxyethylen-9-laurylether, Polyoxypropylen-5-polyoxyethylen-5-laurylether und Mischungen aus diesen beiden Laurylethern.

8. Die Verwendung nach einem der Ansprüche 1 bis 7,
wobei der bzw. die alkoxylierten Monoalkohole in einem Gemisch verwendet werden, das zusätzlich Polyoxyethylen-7-laurylether und/oder Polyoxyethyleneglycerol-tri-hydroxystearat 40 enthält.

9. Eine Zusammensetzung enthaltend
den Solubilisator wie definiert in einem der Ansprüche 1 bis 8 und mindestens einen pharmazeutischen Wirkstoff,
wobei der pharmazeutische Wirkstoff bevorzug eine Löslichkeit in Wasser von höchstens 20, bevorzugt höchstens 10, bevorzugt höchstens 1, und insbesondere höchstens 0,5, g pro Liter bei 25 °C hat.

10. Die Zusammensetzung nach Anspruch 9 weiterhin enthaltend Wasser.

11. Die Zusammensetzung nach einem der Ansprüche 9 bis 10 enthaltend
5 bis 50 Gew.-% Solubilisator, 0,01 bis 15 Gew.-% des mindestens einen pharmazeutischen Wirkstoffes,
0 bis 95 Gew.-% Wasser und 0 bis 90 Gew.-% andere pharmazeutisch akzeptable Komponenten, insbesondere pharmazeutisch akzeptable Hilfs- und/oder Zusatzstoffe.

12. Die Zusammensetzung nach einem der Ansprüche 9 bis 10 zur Anwendung in einem Verfahren zur therapeutischen, inklusive prophylaktischen, Behandlung des menschlichen oder tierischen Körpers.

13. Die Zusammensetzung nach einem der Ansprüche 9 bis 11,
wobei der pharmazeutische Wirkstoff Diazepam ist,
und wobei die Zusammensetzung zur Anwendung als Schlaf- oder Beruhigungsmittel in einem Verfahren zur therapeutischen, inklusive prophylaktischen, Behandlung des menschlichen oder tierischen Körpers bestimmt ist.

14. Die Zusammensetzung nach einem der Ansprüche 9 bis 11,
wobei der pharmazeutische Wirkstoff Erythromycin ist,
und wobei die Zusammensetzung zur Anwendung als Antibiotikum in einem Verfahren zur therapeutischen, inklusive prophylaktischen, Behandlung des menschlichen oder tierischen Körpers bestimmt ist.

15. Die Zusammensetzung nach einem der Ansprüche 9 bis 11,
wobei der pharmazeutische Wirkstoff Itraconazol ist,
und wobei die Zusammensetzung zur Anwendung in einem Verfahren zur therapeutischen, inklusive prophylaktischen, Behandlung des menschlichen oder tierischen Körpers gegen klimakterische Beschwerden oder gegen Osteoporose bestimmt ist.
